# FASCICULE DE BREVET EUROPEEN

(11) **EP 0 958 814 B1**
(45) Date de publication et mention de la délivrance du brevet: **14.05.2003**
(21) Numéro de dépôt: 99400674.0
(22) Date de dépôt: 19.03.1999
(51) Int. Cl.: A61K 9/70, A61K 7/48, A61P 17/00

(54) **Patch à effet thermique et son utilisation**
Thermisch wirkende Pflaster und seiner Verwendung
Patch having a thermal effect and its use

(30) Priorité: 24.03.1998 FR 9803588
(43) Date de publication de la demande: 24.11.1999
(73) Titulaire: L'OREAL, 75008 Paris (FR)
(72) Inventeur: Gueret, Jean-Louis, 75016 Paris (FR)
(74) Mandataire: Leszczynski, André

(56) Documents cités:
- WO-A-86/00536
- DATABASE WPI Section Ch, Week 9028 Derwent Publications Ltd., London, GB; Class A94, AN 90-214636 XP002088451 & JP 02 145854 A (FUTABA SHOKAI) , 5 juin 1990

## Description

La présente invention concerne un patch destiné à être appliqué temporairement sur la peau pour exercer sur celle-ci une action cosmétique et/ou un traitement pharmaceutique.

La demande de brevet japonais JP 02 145854 décrit un tissu recouvert d'une couche de métal, permettant de réfléchir le rayonnement infra rouge du corps humain.

La demande internationale WO 86/00536 décrit un bandage permettant la libération contrôlée de médicaments, constitué d'une couche en aluminium, du médicament à administrer et d'une couche d'adhésif.

On connaît des patchs comportant une feuille de support revêtue sur une face d'une couche formant réservoir appelée matrice, contenant une ou plusieurs substances actives destinées à diffuser dans la peau et/ou à agir sur celle-ci.

L'invention concerne plus particulièrement un patch dans lequel la matrice est réalisée dans un matériau présentant des propriétés adhésives intrinsèques.

Le choix de ce matériau soulève des difficultés, à savoir :
- il doit pouvoir contenir la ou les substances actives destinées à agir sur la peau,
- son adhérence sur la peau ne doit pas être trop forte, notamment s'il est d'application répétée, sous peine d'irriter la région du corps sur laquelle le patch est appliqué et de rendre l'enlèvement de ce dernier douloureux,
- son adhérence ne doit pas non plus être trop faible, sous peine de ne pas pouvoir adhérer à la peau si celle-ci est humide ou le devient, par exemple à cause de la transpiration,
- il doit être suffisamment flexible pour permettre au patch d'épouser le relief de la région du corps sur laquelle il est appliqué,
- il doit rester sur la feuille de support lors du retrait du patch,
- enfin, il doit pouvoir permettre l'extraction des impuretés qui se trouvent à la surface de la peau, notamment le sébum ou la transpiration.

Malgré ces difficultés, quelques matériaux ont pu être proposés pour réaliser la matrice, mais ceux-ci ne conviennent pas forcément à toutes les substances actives que l'on souhaite pouvoir y incorporer.

Les brevets français 2 738 744 ou européen 0 309 309 enseignent notamment d'utiliser pour constituer la matrice des matériaux hydrophobes ou hydrosolubles.

Le matériau hydrosoluble décrit dans le brevet européen 0 309 309 est relativement peu pratique d'emploi car il ne présente pas les propriétés adhésives requises avant l'application sur la peau et nécessite l'humidification préalable de celle-ci.

La présente invention vise à proposer un nouveau patch dont le matériau constituant la matrice répond aux exigences rappelées plus haut et dont la diffusion dans la peau et/ou l'action sur celle-ci de la ou des substances actives contenues à l'intérieur est améliorée.

Le patch selon l'invention se caractérise par le fait qu'il comporte
- une couche réfléchissante vis-à-vis du rayonnement infrarouge libéré par le corps humain, et entre cette couche et ladite face du patch destinée à être appliquée sur la peau,
- une matrice réalisée dans un matériau ayant des propriétés auto-adhésives et comprenant au moins un polymère acrylique ou vinylique dont les propriétés adhésives augmentent avec la température dans la gamme 20-40°C, et
- dans ladite matrice, au moins une substance active ayant un effet sur la peau.

Grâce à l'invention, il se produit un accroissement rapide de la température à l'interface entre la peau et le patch, du fait que le rayonnement thermique libéré par le corps est réfléchi par la couche réfléchissante.

Cela peut avoir de multiples effets avantageux, et notamment :
- accroître notablement la température de la matrice et, compte-tenu du choix particulier du matériau utilisé pour la réaliser, renforcer son adhérence sur la peau,
- favoriser la diffusion de la ou des substances actives au sein de la matrice,
- accroître la circulation sanguine sous le patch, ce qui peut s'avérer favorable à la pénétration et/ou à l'action de cette ou ces substances actives.

Dans une réalisation particulière de l'invention, la matrice est solidaire d'une feuille de support et la couche réfléchissante est située sur cette feuille de support.

De préférence, la couche réfléchissante est alors formée par la métallisation d'au moins une face de ladite feuille de support.

Dans une réalisation particulière de l'invention, le patch comporte en outre une structure perméable telle qu'un filet, noyée au moins partiellement dans la matrice de manière à modifier son pouvoir adhésif global.

Dans une réalisation particulière, le patch comporte en outre une couche apte à capter l'énergie thermique du rayonnement extérieur.

L'invention a encore pour objet une utilisation telle que définie dans la revendication 11.

D'autres caractéristiques et avantages de la présente invention ressortiront à la lecture de la description détaillée qui va suivre, d'un exemple de réalisation de l'invention, et à l'examen du dessin annexé sur lequel :
- la figure 1 est une vue schématique en coupe d'un patch conforme à un exemple de réalisation de l'invention, et
- la figure 2 représente le patch de la figure 1 appliqué sur la peau.

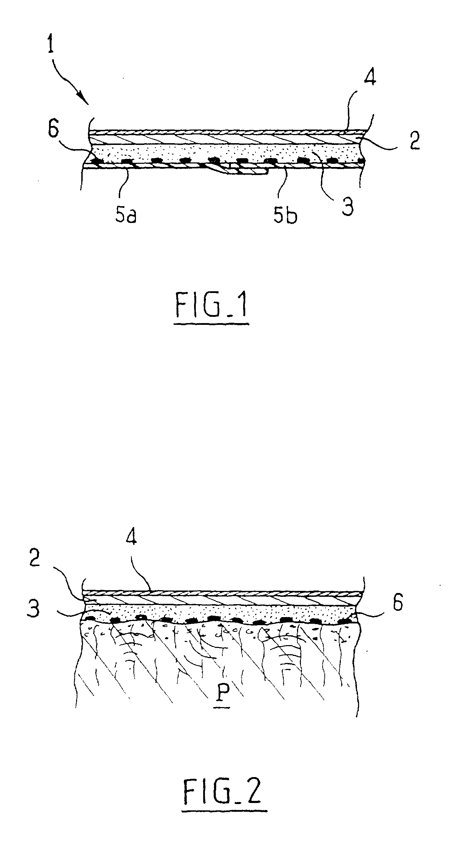

On a représenté sur la figure 1 un patch 1 conforme à un exemple de réalisation de l'invention, comportant une feuille de support 2 revêtue sur une face d'une matrice 3 réalisée dans un matériau ayant des propriétés auto-adhésives.

La feuille de support 2 peut avoir subi un traitement corona visant à améliorer l'adhérence de la matrice 3.

La feuille de support 2 peut être réalisée dans un matériau souple thermiquement isolant, par exemple du polyester, polyéthylène ou polypropylène.

D'une manière générale, la feuille de support 2 peut être réalisée dans tout autre matériau approprié, occlusif ou non.

La matrice 3 est revêtue, sur sa face destinée à être appliquée sur la peau, d'une pellicule de protection amovible, par exemple en polyéthylène siliconé de 50 µm d'épaisseur.

Cette pellicule de protection comporte de préférence, comme représenté sur la figure 1, deux parties 5a, 5b qui se chevauchent dans la région médiane du patch, de manière à permettre à l'utilisateur de les retirer sans toucher des doigts la matrice 3, donc sans lui faire perdre son pouvoir adhésif.

Le patch 1 comporte également une couche réfléchissante 4 destinée à réfléchir le rayonnement thermique émis par le corps de l'utilisateur et permettre ainsi à la matrice 3 d'être rapidement portée à la température de celui-ci.

Dans l'exemple de réalisation décrit, la couche réfléchissante 4 est réalisée par la métallisation de la face extérieure de la feuille de support 2, l'épaisseur de la feuille de support ainsi métallisée étant comprise entre 10 et 120 µm.

Le métal déposé peut être de l'aluminium, de l'argent ou de l'or, l'épaisseur déposée étant choisie de telle sorte que la couche réfléchissante 4 présente l'émissivité lui permettant de renvoyer notablement le rayonnement thermique émis par l'utilisateur vers la matrice 3.

L'épaisseur de métal déposée n'altère pas la flexibilité de la feuille de support 2.

La feuille de support 2 et la matrice 3 viennent directement au contact l'une de l'autre sur toute la surface de leurs faces en regard, et le rayonnement thermique ayant traversé la matrice est directement renvoyé dans cette dernière par la couche réfléchissante.

La totalité de la couche réfléchissante est utile pour renvoyer le rayonnement thermique vers la matrice adhésive.

Dans une variante non représentée, la feuille de support 2 est constituée par une feuille de métal, notamment de l'aluminium.

Dans une autre variante non représentée, la couche réfléchissante présente des ondulations qui peuvent résulter par exemple du fait que la feuille de support 2 est gaufrée.

Il peut en outre s'avérer avantageux de colorer d'une couleur foncée la matrice 3 et/ou la feuille de support 2, de manière à permettre à l'utilisateur, après retrait du patch 1, d'observer par contraste la quantité et/ou la nature des impuretés retirées. L'utilisateur peut alors déterminer si une nouvelle application est nécessaire et également le cas échéant, si la fréquence et/ou la nature du traitement doivent être modifiées.

Le matériau utilisé pour réaliser la matrice 3 comporte un ou plusieurs polymères acryliques ou vinyliques dont l'adhérence (mesurée parallèlement aux surfaces en contact) est de préférence comprise entre 50 et 800 g/cm² et augmente avec la température dans la gamme 20 - 40°C.

L'épaisseur de la matrice 3 est par exemple comprise entre 0,1 et 3 mm.

Une structure perméable telle qu'un filet 6 peut être utilisée pour modifier le pouvoir adhésif global de la matrice 3 comme c'est le cas dans l'exemple représenté sur les figures.

Grâce à la présence de cette structure perméable, on peut utiliser pour réaliser la matrice un matériau qui, en l'absence de la structure perméable, adhérerait trop fortement à la peau pour convenir à la réalisation du patch recherché.

En déposant comme représenté, la structure perméable à la surface de la matrice, on réduit la surface de celle-ci au contact de la peau à une multitude de zones élémentaires de faible section, de sorte que l'adhérence globale sur la peau du matériau constituant la matrice devient compatible avec son utilisation dans un patch.

Par le choix de la structure perméable utilisée, notamment de la section de ses ajours, on peut facilement diminuer ou accroître l'adhérence sur la peau.

Dans une variante non représentée, on peut utiliser une structure perméable noyée dans la matrice de manière à agir sur le pouvoir adhésif global du patch.

La structure perméable utilisée permet le cas échéant la diffusion de la ou des substances actives contenues dans la matrice vers la surface de la peau.

Dans une autre variante non représentée, on pourrait utiliser comme structure perméable non pas un filet mais un non-tissé, au travers duquel peuvent diffuser le cas échéant une ou plusieurs substances contenues dans la matrice.

Ce non tissé est positionné au sein de la matrice de manière à modifier le pouvoir adhésif global de celle-ci et obtenir l'adhérence recherchée.

L'adhérence de la matrice pourrait encore être modifiée en réalisant des reliefs à sa surface.

Ces reliefs peuvent être obtenus au moyen d'une pellicule de protection gaufrée qui, après enlèvement, laisse sur la matrice les reliefs voulus.

La matrice 3 comporte une ou plusieurs substances actives ayant un effet sur la peau telles que par exemple des agents anti-oxydants, anti-radicaux libres, hydratants, dépigmentants, liporégulateurs, anti-acnéiques, anti-séborrhéiques, anti-vieillissement, adoucissants, anti-rides, kératolitiques, anti-inflammatoires, rafraîchissants, cicatrisants, protecteurs vasculaires, anti-bactériens, anti-fongiques, anti-perspirants, déodorants, conditionneurs de la peau, insensibilisants, immunomodulateurs et nourrissants, les absorbeurs d'humidité (coton, polyacrylate) et de sébum (Orgasol).

Le patch selon l'invention peut être fabriqué en enduisant avec le matériau destiné à constituer la matrice 3, alors imbibé d'un ou plusieurs solvants, une bande de transport résistant à la température, puis en faisant passer le tout dans un four destiné à évaporer le ou les solvants précités.

A la sortie du four la bande de transport est séparée de la matrice 3 et celle-ci est appliquée sur la feuille de support 2 métallisée, l'ensemble étant ensuite calandré.

Le patch 1 est de préférence offert à l'utilisateur à l'état pré-découpé, de manière à épouser la forme de la région du corps à traiter, sa taille étant comprise par exemple entre 1 et 30 cm².

Le patch est avantageusement conditionné dans un sachet de protection formé de deux feuilles d'un complexe papier/film en matière plastique étanche, par exemple du polypropylène, le papier étant revêtu d'un adhésif scellable à froid.

Les feuilles sont scellées autour du patch par contact des faces enduites d'adhésif.

Un tel conditionnement présente l'avantage de mettre à l'abri de l'air le patch et d'améliorer sa conservation.

Pour utiliser le patch, l'utilisateur retire la pellicule de protection 5a,5b et applique la matrice 3 sur la peau P, comme illustré sur la figure 2.

Le rayonnement thermique émis par le corps humain est réfléchi par la couche réfléchissante 4 vers la peau P, ce qui provoque une augmentation rapide de la température de la matrice 3 qui atteint la température du corps ou une température proche.

Il en résulte un accroissement de l'adhérence de la matrice sur la peau, une meilleure diffusion le cas échéant de la ou des substances actives contenues dans la matrice 3. et une meilleure pénétration de ces dernières dans la peau, du fait notamment que la chaleur réfléchie par le patch provoque une dilatation des vaisseaux sanguins sous la matrice 3.

La durée d'application est comprise par exemple entre 30 secondes et 5 minutes, et de préférence entre 1 mn et 5 mn.

Des essais ont été réalisés avec un patch réalisé en revêtant une feuille de support en polyester métallisé de 20 µm d'épaisseur avec une matrice comportant un adhésif acrylique en base solvant (éthyle acétate hexane éthanol), auto-réticulant, sensible à la pression, commercialisé sous la dénomination AGXL par la société MAPEI.

La quantité d'adhésif mesurée à sec déposé sur la feuille de support est de 40 g/m².

La matrice 3 comporte également 1,5% en poids d'acide Kojique, 5% de poudre d'Orgasol, 1% d'essences cicatrisantes de géranium et 1% de pigments violets vendus sous la référence DC violet 2K7014 par la Société RDF Chimie.

L'adhérence initiale de l'adhésif utilisé est de 100 g/cm² environ et son pouvoir adhésif (TAC) après un temps de pose suffisant est de l'ordre de 300 g/cm².

Le fait d'utiliser une couche réfléchissante conformément à l'invention permet de concentrer la chaleur et de réduire de manière notable la durée nécessaire pour que l'adhésif atteigne un pouvoir adhésif proche du pouvoir adhésif maximum.

## Revendications

1. Patch destiné à être appliqué par une face sur la peau, **caractérisé par le fait qu'**il comporte
. une couche réfléchissante (4) vis-à-vis du rayonnement infrarouge libéré par le corps humain, et entre cette couche réfléchissante et ladite face destinée à être appliquée sur la peau.
. une matrice (3) réalisée dans un matériau ayant des propriétés auto-adhésives et comprenant au moins un polymère acrylique ou vinylique dont les propriétés adhésives augmentent avec la température dans la gamme 20 - 40°C, et
. dans ladite matrice (3), au moins une substance active ayant un effet sur la peau.

2. Patch selon la revendication 1, **caractérisé par le fait que** la matrice (3) est solidaire d'une feuille de support (2) et **par le fait que** ladite couche réfléchissante (4) est située sur cette feuille de support (2).

3. Patch selon la revendication 2, **caractérisé par le fait que** ladite couche réfléchissante (4) est formée par la métallisation d'au moins une face de la feuille de support (2).

4. Patch selon la revendication 3, **caractérisé par le fait que** la couche réfléchissante présente des ondulations.

5. Patch selon l'une quelconque des revendications précédentes, **caractérisé par le fait que** la matrice est revêtue avant l'utilisation par une pellicule de protection amovible comportant deux parties (5a,5b) se chevauchant.

6. Patch selon l'une quelconque des revendications précédentes, **caractérisé par le fait qu'**il comporte en outre une structure perméable telle qu'un filet (6), noyée au moins partiellement dans la matrice de manière à modifier son pouvoir adhésif global.

7. Patch selon l'une quelconque des revendications précédentes, **caractérisé par le fait que** la matrice présente des reliefs sur sa face destinée à venir au contact de la peau.

8. Patch selon l'une quelconque des revendications précédentes, **caractérisé par le fait qu'**il comporte une couche apte à capter l'énergie thermique d'un rayonnement extérieur.

9. Patch selon l'une quelconque des revendications précédentes, **caractérisé par le fait qu'**il est conditionné dans un sachet de protection formé de deux feuilles d'un complexe papier/film en matière plastique étanche, le papier étant revêtu d'un adhésif scellable à froid, les feuilles étant scellées autour du patch par contact des faces enduites d'adhésif.

10. Patch selon l'une quelconque des revendications précédentes, **caractérisé par le fait que** la totalité de la couche réfléchissante est utile pour renvoyer le rayonnement thermique vers la matrice adhésive.

11. Utilisation d'une matrice contenant une substance active ayant un effet sur la peau pour la fabrication d'un patch (1) tel que défini dans l'une quelconque des revendications précédentes, permettant de réfléchir localement le rayonnement thermique émis par le corps humain et favoriser par un effet thermique la pénétration de la substance active dans la peau.

12. Utilisation selon la revendication précédente, **caractérisée par le fait que** la durée d'application du patch est comprise entre 30 secondes et 5 minutes.

## Patentansprüche

1. Pflaster, das dazu bestimmt ist, mit einer Seite auf die Haut aufgelegt zu werden, **dadurch gekennzeichnet, dass** es umfasst:
- eine Schicht (4), die gegenüber der vom menschlichen Körper abgegebenen Infrarotstrahlung reflektierend ist, und zwischen dieser reflektierenden Schicht und dieser Seite, die dazu bestimmt ist, auf die die Haut aufgelegt zu werden,
- eine Matrize (3), die aus einem Werkstoff besteht, der selbsthaftende Eigenschaften besitzt und mindestens ein Acryl- oder Vinylpolymer umfasst, dessen Hafteigenschaften mit der Temperatur im Bereich von 20 bis 40°C zunehmen, und
- in dieser Matrize (3) mindestens eine aktive Substanz, die eine Wirkung auf die Haut hat.

2. Pflaster nach Anspruch 1, **dadurch gekennzeichnet, dass** die Matrize (3) mit einer Trägerfolie (2) fest verbunden ist und dass die reflektierende Schicht (4) auf dieser Trägerfolie (2) angeordnet ist.

3. Pflaster nach Anspruch 2, **dadurch gekennzeichnet, dass** diese reflektierende Schicht (4) durch die Metallisierung mindestens einer Seite der Trägerfolie (2) gebildet wird.

4. Pflaster nach Anspruch 3, **dadurch gekennzeichnet, dass** die reflektierende Schicht Wellungen aufweist.

5. Pflaster nach einem der vorhergehenden Ansprüche, **dadurch gekennzeichnet, dass** die Matrize vor der Verwendung mit einer abnehmbaren Schutzhaut bedeckt ist, die zwei sich überlappende Teile (5a, 5b) aufweist.

6. Pflaster nach einem der vorhergehenden Ansprüche, **dadurch gekennzeichnet, dass** es außerdem eine durchlässige Struktur wie ein Netz (6) aufweist, die mindestens teilweise in die Matrize eingebettet ist, so dass ihr Gesamthaftvermögen verändert wird.

7. Pflaster nach einem der vorhergehenden Ansprüche, **dadurch gekennzeichnet, dass** die Matrize auf der Seite, die dazu bestimmt ist, mit der Haut in Kontakt zu kommen, Erhebungen aufweist.

8. Pflaster nach einem der vorhergehenden Ansprüche, **dadurch gekennzeichnet, dass** es eine Schicht aufweist, die in der Lage ist, die thermische Energie einer äußeren Strahlung einzufangen.

9. Pflaster nach einem der vorhergehenden Ansprüche, **dadurch gekennzeichnet, dass** es in einem Schutzbeutel verpackt ist, der von zwei Folien eines Komplexes Papier/dichte Kunststofffolie gebildet wird, wobei das Papier mit einem kaltsiegelbaren Klebstoff bedeckt ist, wobei die Folien um das Pflaster herum durch Kontakt der mit Klebstoff beschichteten Seiten versiegelt sind.

10. Pflaster nach einem der vorhergehenden Ansprüche, **dadurch gekennzeichnet, dass** die Gesamtheit der reflektierenden Schicht nützlich ist, um die Wärmestrahlung zur haftenden Matrize zurückzusenden.

11. Verwendung einer Matrize, die eine aktive Substanz mit einer Wirkung auf die Haut enthält, für die Herstellung eines Pflasters (1) nach einem der vorhergehenden Ansprüche, das gestattet, die von dem menschlichen Körper abgegebene Wärmestrahlung örtlich zu reflektieren und das Eindringen der aktiven Substanz in die Haut durch einen thermischen Effekt zu begünstigen.

12. Verwendung nach dem vorhergehenden Anspruch, **dadurch gekennzeichnet, dass** die Anwendungsdauer des Pflasters zwischen 30 Sekunden und 5 Minuten beträgt.

## Claims

1. Patch intended to be applied via one face to the skin, **characterized in that** it comprises:
• a layer (4) that reflects the infrared radiation released by the human body, and, between this reflective layer and the said face intended to be applied to the skin,
• a matrix (3) made of a material that has self-adhesive properties and comprising at least one acrylic or vinyl polymer whose adhesive properties increase with temperature in the range 20-40°C, and
• in the said matrix (3), at least one active substance that has an effect on the skin.

2. Patch according to Claim 1, **characterized in that** the matrix (3) is securely fastened to a support sheet (2) and **in that** the said reflective layer (4) is located on this support sheet (2).

3. Patch according to Claim 2, **characterized in that** the said reflective layer (4) is formed by metallization of at least one face of the support sheet (2).

4. Patch according to Claim 3, **characterized in that** the reflective layer has undulations.

5. Patch according to any one of the preceding claims, **characterized in that** the matrix is coated before use with a removable protective film comprising two overlapping portions (5a, 5b).

6. Patch according to any one of the preceding claims, **characterized in that** it also comprises a permeable structure such as a net (6), at least partially buried in the matrix so as to modify its overall adhesive power.

7. Patch according to any one of the preceding claims, **characterized in that** the matrix has reliefs on its face intended to come into contact with the skin.

8. Patch according to any one of the preceding claims, **characterized in that** it comprises a layer capable of capturing the thermal energy of an external radiation.

9. Patch according to any one of the preceding claims, **characterized in that** it is packaged in a protective sachet formed from two sheets of a leaktight paper/plastic film complex, the paper being coated with a cold-sealable adhesive, the sheets being sealed around the patch by contact with the faces coated with adhesive.

10. Patch according to any one of the preceding claims, **characterized in that** all of the reflective layer is useful for conveying the thermal radiation towards the adhesive matrix.

11. Use of a matrix containing an active substance that has an effect on the skin, for the manufacture of a patch (1) as defined in any one of the preceding claims, making it possible locally to reflect the thermal radiation emitted by the human body and to promote, by means of a thermal effect, the penetration of the active substance into the skin.

12. Use according to the preceding claim, **characterized in that** the duration of application of the patch is between 30 seconds and 5 minutes.
